(19) **Europäisches Patentamt**
**European Patent Office**
**Office européen des brevets**

(11) **EP 2 460 876 B1**

(12) **EUROPEAN PATENT SPECIFICATION**

(45) Date of publication and mention
of the grant of the patent:
**04.09.2013 Bulletin 2013/36**

(21) Application number: **10803953.8**

(22) Date of filing: **30.07.2010**

(51) Int Cl.:
*C12N 5/0775* (2010.01)   *G01N 33/53* (2006.01)
*G06F 19/00* (2011.01)   *C12Q 1/68* (2006.01)

(86) International application number:
**PCT/ES2010/070534**

(87) International publication number:
**WO 2011/012764 (03.02.2011 Gazette 2011/05)**

(54) **METHOD FOR IDENTIFYING SENESCENT MESENCHYMAL STEM CELLS**

VERFAHREN ZUR IDENTIFIKATION ALTERNDER MESENCHYMALER STAMMZELLEN

MÉTHODE D'IDENTIFICATION DE CELLULES SOUCHES MÉSENCHYMATEUSES SÉNESCENTES

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **30.07.2009 ES 200930540**

(43) Date of publication of application:
**06.06.2012 Bulletin 2012/23**

(73) Proprietor: **Centro Nacional de Investigaciones Cardiovasculares (CNIC)**
**28029 Madrid (ES)**

(72) Inventors:
• **SAMPER RODRÍGUEZ, Enrique**
**E-28029 Madrid (ES)**
• **ESTRADA RODRÍGUEZ, Juan Camilo**
**E-28029 Madrid (ES)**
• **BERNAD MIANA, Antonio**
**E-28029 Madrid (ES)**

(74) Representative: **Pons Ariño, Angel**
**Pons Patentes y Marcas Internacional, S.L.**
**Glorieta Rubén Dario 4**
**28010 Madrid (ES)**

(56) References cited:
**WO-A1-2009/073518**

• **WAGNER WOLFGANG ET AL: "Replicative Senescence of Mesenchymal Stem Cells: A Continuous and Organized Process", PLOS ONE, vol. 3, no. 5, May 2008 (2008-05), XP002686801, ISSN: 1932-6203**

• **RYU E ET AL: "Identification of senescence-associated genes in human bone marrow mesenchymal stem cells", BIOCHEMICAL AND BIOPHYSICAL RESEARCH COMMUNICATIONS, ACADEMIC PRESS INC. ORLANDO, FL, US, vol. 371, no. 3, 4 July 2008 (2008-07-04), pages 431-436, XP022670717, ISSN: 0006-291X, DOI: 10.1016/J.BBRC.2008.04.111 [retrieved on 2008-04-29]**

• **CHRISTINE FEHRER ET AL: "Reduced oxygen tension attenuates differentiation capacity of human mesenchymal stem cells and prolongs their lifespan", AGING CELL, BLACKWELL PUBLISHING, GB, vol. 6, no. 6, 1 December 2007 (2007-12-01), pages 745-757, XP002643661, ISSN: 1474-9718, DOI: 10.1111/J. 1474-9726.2007.00336.X [retrieved on 2007-08-13]**

• **BONAB MANDANA MOHYEDDIN ET AL: "Aging of mesenchymal stem cell in vitro", BMC CELL BIOLOGY, BIOMED CENTRAL, LONDON, GB, vol. 7, no. 1, 10 March 2006 (2006-03-10), page 14, XP021014091, ISSN: 1471-2121, DOI: 10.1186/1471-2121-7-14**

• **LECHEL, A. ET AL.: 'The cellular level of telomere dysfunction determines induction of senescence or apoptosis in vivo.' EMBO REPORTS. vol. 6, no. 3, March 2005, pages 275 - 281, XP008151458**

• **CHAMBERS, S.M. ET AL.: 'Aging hematopoietic stem cells decline in function and exhibit epigenetic dysregulation.' PLOS BIOLOGY. vol. 5, no. 8, August 2007, page E201., XP008151459**

**(Cont. next page)**

- WALL, LB. ET AL.: 'Fibroblast dysfunction is a key factor in the non-healing of chronic venous leg ulcers.' THE JOURNAL OF INVESTIGATIVE DERMATOLOGY. vol. 128, no. 10, October 2008, pages 2526 - 2540, XP008151460
- MAEHARA, K. ET AL.: 'CENP-A reduction induces a p53-dependent cellular senescence response to protect cells from executing defective mitoses.' MOLECULAR AND CELLULAR BIOLOGY. vol. 30, no. 9, 16 February 2010, pages 2090 - 2104, XP008151461
- FUNAYAMA, R. ET AL.: 'Loss of linker histone H1 in cellular senescence.' THE JOURNAL OF CELL BIOLOGY vol. 175, no. 6, 18 December 2006, pages 869 - 880, XP008151462

**Description**

The present invention is in the field of cellular biology and genetics and refers to a method for identifying senescent mesenchymal stem cells growing in *in vitro* culture and comprises: measuring the length of the telomeres in the chromosomes, determining the ploidy levels in the cell, detecting the presence of multipolar mitosis and determining the level of expression of the genes SCIN, AKAP9, EDN-1, CXCL1, CXCL12 and/or CD70. This method can be of use for carrying out studies of genetic stability in mesenchymal stem cell cultures, enabling identification and selection of the most stable and appropriate cells to be employed in cell therapy.

BACKGROUND OF THE INVENTION

Human mesenchymal stem cells (hMSC) has been proposed in recent years as a powerful tool for cell therapy because of their high potential for proliferating and differentiating into cells derived from the mesoderm (osteocytes, chondrocytes and adipocytes). This enables their use in the treatment of some pathologies associated with chronic inflammation, ageing, autoimmune diseases and pathologies associated with traumas such as, for example, graft-versus-host disease, fistulas related to Crohn's disease, rheumatoid arthritis, myocardial infarction and degenerative conditions of the cartilage and bone.

However, although there are sources of MSCs available in different tissues, their numbers in the body are low. The majority of cell therapy protocols use between 10-50 million hMSC per treatment, so these cells must be expanded in *in vitro* culture for 4 to 8 weeks before implantation. In addition, these cultures are often carried out in pre-oxidising conditions (20 % $O_2$) and increased concentrations of saline and glucose where the cells may suffer mutations and chromosomal abnormalities that put their biosafety at risk if they are to be used in the clinic. The long periods of culture and oxidative damage contribute to cell senescence and genetic instability, which may change the properties of the cells, limiting their biomedical utility.

Senescence is mainly mediated by the activation of tumour-suppressor genes that stop the cell cycle, p15/p16/Rb and p19$^{arf}$/p53/p21 (US2002123526 A1). Senescent cells also over-express markers associated with stress such as SA-β-galactosidase or Lipofuscine (Krzysztof Ksiazek, 2009, Rejuvenation Research, Vol. 12, No. 2: 105-116), as well as programmes that increase the level of proinflammatory cytokines. However, the identification of more specific senescence biomarkers is necessary to detect the level of senescence in mesenchymal stem cell cultures in order to carry out quality controls before their biomedical application.

Senescent cells exhibit alterations in some of their physiological parameters and alter the characteristics of neighbouring cells. Polyploidy (duplications of whole entire set of chromosomes) constitutes a marker of cellular stress in the majority of tissues and it is believed to be a precursor mechanism of aneuploidy, which is considered to be a fundamental factor associated with ageing and with cell transformation because the majority of tumours are aneuploid. Some authors have demonstrated the presence of aneuploidy in mesenchymal stem cells originating from the bone marrow of non-human primates growing in culture (Reza Izadpanah, et al., 2008, Cancer Research, Vol. 68, No. 11: 4229-4238). Therefore, aneuploidy has been proposed as another characteristic of senescent cells (Steven R. Schwarze, et al., 2005, Neoplasia, Vol. 7, No. 9: 816-823). Previous studies have demonstrated that during prolonged cell culture of human embryonic stem cells there is a predisposition for maintenance and selection of specific chromosomal aneuploidies for chromosomes 12, 17, X and 20q, which may possibly provide a selective advantage for propagation of human embryonic stem cells that are maintained indefinitely in culture (Spits, C. et al., 2008, Nature Biotechnology, Vol. 26: 1361-1363).

One of the main mechanisms for the induction of senescence in cells is shortening of the telomeres. Telomeres are the terminal ends of eukaryotic chromosomes and are crucial for maintaining genetic stability: maintenance of length and function of telomeres is a requirement for cell division and for correct chromosomal segregation. The maintenance process in the majority of cells is performed by telomerase reverse transcriptase that adds repetitions to the end of the chromosomes. Senescence of mesenchymal stem cells has been associated with progressive shortening of the telomere ends with successive culture cycles (Melissa A. Baxter, et al., 2004, Stem Cells, 22:675-682).

Another of the approaches investigated for the detection of senescence biomarkers in mesenchymal stem cells in culture is the analysis of genetic expression of the entire transcriptome of these cells in order to identify those genes that are expressed differently in senescent and non-senescent cells. The genes identified for use as senescence biomarkers are mostly related to cell proliferation, response to stress, development, cell cycle, mitosis, replication and DNA repair, etc. (Eunsook Ryu, et al., 2008, Biochemical and Biophysical Research Communications, 371:431-436).

Other studies based on the identification of senescence biomarkers have investigated the impact of senescence on the phenotype, differentiation and global gene expression patterns of MSCs growing in culture. Thus, a shortening of chromosome telomeres and reduced expression of some of the genes involved in DNA replication and repair have been associated with senescence in these cells (Wolfgang Wagner, et al., 2008, PLoS ONE, Vol. 3, No. 5).

So shortening of telomeres, aneuploidy and changes in the gene expression patterns compared to non-senescent cells are characteristics that have been associated with the senescent phenotype. Nevertheless, it is necessary to have available a complete, reliable and reproducible method that analyses all the parameters involved in the development of senescence and that is capable of detecting senescent mesenchymal stem cells in culture. This method will enable analysis of their genetic stability, which is particularly relevant given the important applications of these cells in the field of cell therapy.

DESCRIPTION OF THE INVENTION

The present invention provides a method for identifying senescent mesenchymal stem cells growing in *in vitro* culture comprising: measuring the average length of chromosome telomeres, determining the level of ploidy in the cell, determining the number of multipolar mitoses and determining the level of expression of the genes SCIN (scinderin), EDN-1 (endothelin-1), AKAP9 (A-kinase anchor protein 9; Yotiao), CXCL12, CXCL1 and CD70 involved in control of ploidy and polyploidization. This method can be of use for carrying out studies of genetic stability in mesenchymal stem cell cultures, enabling identification and selection of the most stable and appropriate cells to be employed in cell therapy.

The inventors have carried out an analysis of differential gene expression of mesenchymal stem cells in passage 21 and passage 2 of a culture in the presence of 20 % of $O_2$ to discover which genes are expressed differently in the two populations and if this difference in expression is significant. After undertaking an analysis to identify which genes with changed expression are involved in genetic instability processes, they have shown that there are 69 altered genes involved in cancer processes and the cell cycle. Of all the genes where expression is altered in senescence, a total of 8 were selected with the most significant differential expression that are involved in the maintenance of ploidy levels.

Based on this analysis, it was concluded that senescence of mesenchymal stem cells is related to overexpression of SCIN (scinderin), EDN-1 (endothelin-1) and/or AKAP9 (A-kinase anchor protein 9; Yotiao) and/or with a reduction in the expression of CXCL12, CXCL1 and/or CD70 involved in the control of ploidy and polyploidization. Therefore, these genes are proposed in the present invention as biomarkers of genetic instability in mesenchymal stem cell cultures.

In addition, human MSC exhibit significantly reduced telomere length and increased levels of aneuploidy and multipolar mitosis, which are related to the reduced expression of the genes controlling ploidy. Therefore, the method of the present invention comprises the analysis of all these senescence parameters together in order to detect senescence in MSCs that are being expanded in *in vitro* culture.

Therefore, a first aspect of the invention refers to a method for identifying senescent mesenchymal stem cells, hereinafter referred to as the "method of the invention", comprising:

a. measuring the length of the chromosome telomeres of the mesenchymal stem cell,
b. determining the ploidy level of the mesenchymal stem cell of step (a),
c. analyzing the presence of multipolar mitosis of the mesenchymal stem cell of step (b),
d. analyzing the level of expression of the genes SCIN, EDN-1, AKAP9, CXCL1, CXCL12 and/or CD70 in the mesenchymal stem cell of step (c), and
e. associating the data obtained in steps (a)-(d) with a senescent phenotype.

In a preferred embodiment of this aspect of the invention, the measurement of the length of chromosome telomeres in step (a) is carried out by at least one of the following methods: quantitative FISH or TRAP. In another preferred embodiment, the determination of the ploidy level in step (b) is carried out by hybridisation with specific chromosome probes and subsequently counting their fluorescent signals. In another preferred embodiment, the specific chromosomal probes are CEP probes. In a more preferred embodiment, the CEP probes are specific for chromosomes 8, 10, 11 and/or 17. In a still more preferred embodiment, the CEP probes are specific for chromosome 10.

In the present invention, "mesenchymal stem cells" or "mesenchymal progenitor cells" are understood to be adult stem cells that are distributed in connective tissue of various organs such as the osseous medulla, peripheral blood, umbilical cord, trabecular bone, adipose tissue, synovial tissue, deciduous teeth, skeletal muscle and also some tissues of the foetus, where the characteristic markers include but are not limited to SH2, SH3, SH4, CD10, CD13, CD29, CD44, CD54, CD73, CD90, CD105 and CD166, and are negative for the markers CD31, CD34, CD38, CD40 or CD45. These cells have the capacity to differentiate into multiple cell lines of the mesoderm such as cells of the osteogenic, adipogenic and chondrogenic cell lines.

These mesenchymal stem cells, in order to be applied in cell therapy such as, for example, tissue regeneration, need to be expanded in *in vitro* culture, which provides therapeutically useful cell numbers. However, after a certain number of divisions these cells become senescent and reach limits to their proliferative capacity, their potential for differentiation, their genetic stability, etc. "Senescence" is understood as the phenomenon that induces changes in some parameters of cell physiology, limits their proliferative capacity, replication, differentiation, etc., in addition to changing gene expression patterns. It may be induced by various sources of stress such as ionising radiation, oncogenic activation, telomere shortening, incomplete repair of DNA, chromatin disturbances or oxidative damage and is associated with responses to damage of the DNA. Cells that grow for a long time in *in vitro* culture exhibit senescence.

In order to identify a mesenchymal stem cell as senescent, the method of the invention comprises, firstly, measurement of the length of chromosome telomeres of the cell, because reduction in the telomere length is one of the characteristics that are associated with the senescent phenotype. "Telomeres" are non-coding regions of the DNA that are found at the ends of linear chromosomes. In human cell chromosomes, telomeres are formed by some 2,000 tandem repeats of the telomere sequence "TTAGGG/AATCCC". Telomeres are involved in many cell functions related to chromosomal stability and cell division.

Measurement of telomere length can be carried out by any method known in the state of the art such as, for example but not limited to, *slot-blot,* TRF or analysis of terminal restriction fragments obtained by digestion of DNA with restriction enzymes and subsequent Southern Blot, TRAP or Telomeric Repeat Amplification Protocol, which analyses levels of telomerase activity, quantitative or non-quantitative fluorescence *in situ* hybridisation (FISH) and primed *in situ* synthesis (PRINS). In a preferred embodiment of the first aspect of the invention, measurement of the length of chromosome telomeres is carried out by quantitative FISH and/or TRAP, the protocols of which are described in the examples of the present invention. In the method of the invention, it is not necessary to carry out the two methods, quantitative FISH and TRAP, to measure the telomere length because either of them provides sufficient information of this parameter on its own. However it is preferable to carry out both in order to obtain more complete information because the TRAP method provides evidence on the activity of telomerase, the enzyme that maintains the telomere length. Therefore, in a preferred embodiment, the measurement of chromosome telomere length in step (a) is carried out by quantitative FISH, in another preferred embodiment the measurement of chromosome telomere length is carried out by TRAP, and in a more preferred embodiment, this measurement is carried out by quantitative FISH and TRAP.

Secondly, the method of the invention comprises analysis of the ploidy level of the mesenchymal stem cell. "Ploidy level" is understood to be the number of complete chromosome sets in a cell, for example, 2n, 3n, 4n, etc. Human somatic cells are diploid (2n) and any change in this number of chromosome sets is understood to be "aneuploidy". The determination of the ploidy level in a cell can be carried out by cytogenetic techniques that include, but are not limited to, the use of probes that identify specific and/or repetitive structures of the chromosomes such as, for example but not limited to, FISH with centromere probes such as but not limited to, CEP or LS1 probes, DAPI (4',6-diamidino-2-phenylindole) or chromosome specific probes or by the ImagePath™ DNA Ploidy system. In a preferred embodiment, the determination of ploidy level of the mesenchymal stem cell is carried out by hybridisation with chromosome specific centromere probes, specifically but not limited to, the CEP probes known in the state of the art. These probes can be specific for any of the chromosomes because the fluorescent hybridisation signal of these probes with any chromosome allows obtaining information about the cellular ploidy level. But in the present invention, these are preferably specific for chromosomes 8, 10, 11 and/or 17 because during prolonged cell culture a progressive increase in aneuploidy levels for these four chromosomes (preferably towards an increase in the number of copies) has been demonstrated. This increase in ploidy level is especially marked for the case of chromosome 10, as shown in the examples and figures of the present invention. Therefore, in a preferred embodiment, the determination of the ploidy level in step (b) is carried out by hybridisation with chromosome enumeration probes (CEP) specific for chromosome 10 and subsequently assaying their fluorescent signal.

Subsequent analysis of the probes' fluorescence signal enables visualisation of the number of each of these chromosomes in the cell, so aneuploidy is concluded when there are more or less than two signals per chromosome probe.

The third step of the method of the invention comprises analysing the presence of multipolar mitosis in the cell. "Multipolar mitosis" is understood as that cell division in which the spindle has three or more poles, giving rise to the formation of the corresponding number of daughter cells. Multipolar mitosis can be a cause of polyploidy in cells. Multipolar mitosis analysis can be carried out by, for example but not limited to, any cytological technique known in the state of the art that allows the analysis of the cell nucleus such as, for example but not limited to, staining with anti-tubulin antibodies.

A fourth step of the method of the invention comprises the determination of the level of expression of the genes SCIN (scinderin), EDN-1 (endothelin-1), AKAP9 (A-kinase anchor protein 9; Yotiao), CXCL12 (chemokine ligand 12, stromal cell-derived factor-1), CXCL1 (chemokine ligand 1, Gro-alpha) and/or CD70 (molecule CD70) in mesenchymal stem cells. Analysis of the expression of these genes can be carried out by, for example but not limited to, PCR, RT-PCR, immunohistochemical techniques for the detection of these proteins in the cell, protein microarrays, cDNA microarrays, Western Blot, analysis of their RNA levels in the cell by, for example but not limited to, quantification of signals from fluorescent probes of mRNA, etc. The method of analysis of the expression of these genes carried out in the method of the invention is preferably that described in the examples.

The final step of the method of the invention comprises associating the data obtained from measurement of chromosome telomere length, determination of the ploidy level, analysis of the presence of multipolar mitosis and determination of the level of expression of the previously mentioned genes in the mesenchymal stem cell with a senescent phenotype.

Therefore, in another preferred embodiment of this aspect of the invention, the senescent phenotype in step (e) is characterised by showing:

5

a. a reduction in the chromosome telomere length compared to a non-senescent cell,
b. aneuploidy,
c. multipolar mitosis,
d. an increased expression of the genes SCIN, AKAP9 and/or EDN-1, and/or
e. a reduced expression of the genes CXCL1, CXCL12 and/or CD70.

The reduction in the chromosome telomere length in step (a) can be determined by comparison of the chromosome telomere length of a senescent mesenchymal stem cell to a non-senescent mesenchymal stem cell. Aneuploidy is understood, in the senescent phenotype, as the increase or reduction in the number of copies of chromosomes 8, 10, 11 and/or 17, but not limited to these, compared to the normal number of copies of the same chromosomes in a non-senescent cell, that is, a change in the number of copies of these chromosomes to above or below two, as explained above. Aneuploidy of the senescent phenotype of the invention is characterised by showing an increase in the number of copies of chromosomes 8, 10, 11 and/or 17 and more preferably of chromosome 10, with "increase in the number of copies" being understood to mean the presence of one or more copies of these chromosomes in the cell over their normal number in a non-senescent cell, that is, one or more copies of these chromosomes greater than two.

Multipolar mitosis is understood, in the senescent phenotype, as the presence of cellular divisions in which the spindle has three or more poles.

"Increased expression" is understood to mean that expression in which the levels of the genes SCIN, AKAP9 and EDN-1 are higher than the levels of expression of the same genes in a non-senescent mesenchymal stem cell. Similarly, "reduced expression" is that expression in which the levels of the genes, SCIN, AKAP9 and EDN-1, are below the levels of expression of the same genes in a non-senescent mesenchymal stem cell.

In the present invention, the existence of other biomarkers related to genetic stability and as a consequence to senescence was also determined.

The first group of genes related to genetic stability are, as shown in Table 3 of the example section of the present invention, HIST2H2AC, HIST1H4C, HIST1H2BK, HIST1H4L, HIST1H2BE, HIST1H4B, HIST1H2BO, HIST1H4D, HIST1H2BL, HIST1H2BF, HIST1H2BH, HIST1H3D, HIST1H1A, HIST1H2BI, HIST1H1B, HIST1H2BB, HIST1H2AE, HMGA1, HIST1H2BC, HIST1H1C, HIST1H2BD, HIST1H2BM, HIST1H2BJ, HIST1H2BN, HIST2H2AA4, HIST2H2BE and HIST1H2AD. These genes can be detected individually or in any combination, including the combination of all of them, or of any of them, or in the combination of any biomarker of this group with any biomarker of any of the groups mentioned in the present invention.

Therefore, the senescent phenotype is characterised by showing:

- an increased expression of the genes coding for the histones of family H2B: HIST1H2BK, HIST1H2BE, HIST1H2BO, HIST1H2BL, HIST1H2BF, HIST1H2BH, HIST1H2BI, HIST1H2BB, HIST1H2BC, HIST1H2BD, HIST1 H2BM, HIST1 H2BJ, HIST1 H2BN oHIST1 H2AD, and/or
- a reduced expression of the genes coding for the histones of family H4: HIST1 H4C, HIST1 H4L, HIST1 H4B oHIST1 H4D, and/or
- an increased expression of the genes coding for histones HIST1H3D, HIST1H2AE, HIST1H1C, HIST2H2AA4 or HIST1H2AD and/or
- a reduced expression of the genes coding for HIST2H2AC, HIST1H1A, HIST1H1 B or the non-histone protein HMGA1.

Another group of genes related to chromosomal separation during mitosis are, as shown in Table 4 of the example section of the present invention, CENPM, CENPO, CENPA, CENPN, CENPV, CENPK, CENPF and CENPQ. These genes can be detected individually or in any combination, including the combination of all of them, or of any of them, or in the combination of any biomarker of this group with any biomarker of the any of the groups mentioned in the present invention.

Therefore, the senescent phenotype is characterised by showing:

- a reduced expression of the genes coding for the proteins of the complex associated to the centromere (CENP-NAC): CENPM, CENPO, CENPA, CENPN, CENPV, CENPK and CENPF, and/or
- an increased expression of gene CENPQ.

Another group of genes related to the stability of the centrosome, centromere and kinetochore (subfunctions affecting chromosomal segregation) are, as shown in Table 5 of the example section of the present invention, SPC25, TNFAIP3, GTSE1, HAUS8, CDC45L, SKA1, PLK1, SKA3, KIF22, ESPL1, PXN, BIRC5, BUB1 B, CCNB1, KIFC1, SPAG5, NDC80, HAUS7, EVI5, KIF4A, CDC25B, KIF20A, DYNLT3, sep-07, CEP63, CEP290 and EVI5. These genes can be detected individually or in any combination, including the combination of all of them, or of any of them, or in the combination of any biomarker of this group with any biomarker of the any of the groups mentioned in the present invention.

Therefore, the senescent phenotype is characterised by showing:

- a reduced expression of the genes SPC25, TNFAIP3, GTSE1, HAUS8, CDC45L, SKA1, PLK1, SKA3, KIF22, ESPL1, PXN, BIRC5, BUB1B, CCNB1, KIFC1, SPAG5, NDC80, HAUS7, EVI5, KIF4A, CDC25B and KIF20A, and/or
- an increased expression of the genes DYNLT3, sep-07, CEP63, CEP290 and EVI5.

Another preferred embodiment of the present invention refers to the method for identifying senescent stem cells of the invention, where the levels of expression of the genes HIST1 H4C, HIST1 H4L, HIST1 H1C, CENPM, DYNLT3, SPC25, GTSE1, CDC45L, PLK1 and SKA3 in the mesenchymal stem cell are analysed, and the data obtained are associated with the senescent phenotype.

That is, the determination of the level of expression of the genes HIST1H4C, HIST1H4L, HIST1H1C, CENPM, DYNLT3, SPC25, GTSE1, CDC45L, PLK1 and SKA3 is carried out in step (d) of the method of the invention, that is, in the step in which the level of expression of the genes SCIN, EDN-1, AKAP9, CXCL1, CXCL12 and CD70 is determined in the mesenchymal stem cell of step (c) and subsequently the data obtained in steps (a)-(d) are associated with a senescent phenotype.

A more preferred embodiment of the present invention refers to identifying senescent mesenchymal stem cells of the invention where the senescent phenotype is characterised by showing:

a. an increased expression of the genes HIST1H1 C and DYNLT3, and
b. a reduced expression of the genes HIST1H4C, HIST1H4L, CENPM, SPC25, GTSE1, CDC45L, PLK1 and SKA3.

In addition to the genes SCIN, EDN-1, CXCL1, CXCL12 and CD70, a further 10 genes were selected that increase the efficacy of the method for identifying senescent cells of the invention. These 10 genes were selected taking into account their change in expression and statistical significance (Tables 3, 4 and 5). These genes were selected from a new array and are also included in the three tables of genes previously mentioned. A description of each of the selected genes is given below:

HIST1H4C HISTONE CLUSTER 1, H4c (Table 3).
HIST1H4L HISTONE CLUSTER 1, H4l (Table 3).
HIST1H1C HISTONE CLUSTER 1, H1c (Table 3).
CENPM CENTROMERE PROTEIN M (Table 4).
DYNLT3 Dynein light chain Tctex-type 3 (Table 5).
SPC25 SPC25, NDC80 kinetochore complex component, homolog (*S. cerevisiae*) (Table 5).
GTSE1 G2 and S phase-expressed protein 1 (Table 5).
CDC45L CDC45 cell division cycle 45-like (*S. cerevisiae*) (Table 5).
PLK1 Polo-like kinase 1 (*Drosophila*) (Table 5).
SKA3 Spindle and kinetochore associated complex subunit 3 (Table 5).

In another preferred embodiment, mesenchymal stem cells are grown in *in vitro* culture. In another preferred embodiment, mesenchymal stem cells come from a human.

The method of the invention can be applied to, but without being limited, mesenchymal cells that are growing *in vitro,* so that it can serve both for identifying senescence in an isolated mesenchymal cell and for identifying senescence in a culture of mesenchymal stem cells that preferably are to be used in cell therapy.

Therefore the method of the invention can be of use for carrying our genetic stability studies in cultures of mesenchymal stem cells. In a preferred embodiment of this aspect of the invention, the mesenchymal stem cell cultures are for use in cell therapy.

Senescence and shortening of telomeres can lead to an increase in genetic instability. Thus, identification of senescent mesenchymal stem cells in an *in vitro* culture can provide information on the degree of genetic stability of these cells in culture, a parameter that is important for determining the quality of the cells that are to be used for therapeutic ends. "Genetic stability" is understood to be the state in which the cells do not show significant changes in their genetic expression profiles compared to the expression profile expected in cells of the same lineage; they do not exhibit an increased number of mutations, changes in their ploidy level, increased number of multipolar mitoses, significant changes in their telomere length, etc.

"Cell therapy" is understood to be the use of live cells, either autologous (originating from the patients themselves) or allogeneic (from another human being) or xenogeneic (from animals), where the biological characteristics have been substantially altered as a result of manipulation in order to obtain a therapeutic, diagnostic or preventative effect, by metabolic, pharmacological or immunological means.

Another aspect of the invention refers to a kit for identifying senescent mesenchymal stem cells, hereinafter the "kit of the invention", that comprises suitable means for carrying out the method of the invention. In another preferred embodiment, the kit of the invention comprises: PNA probes, CEP probes and Taqman probes that are specific for the genes SCIN, AKAP9, EDN-1, CXCL1, CXCL12 and CD70. In a more preferred embodiment, the CEP probes are specific for chromosomes 8, 10, 11 and/or 17.

"PNA probes" are understood to be telomere Peptide Nucleic Acid LL(CCCTAA)3 probes labelled with, for example but not limited to, Cy3. These are hybrid nucleic acid and protein probes designed for the detection of specific sequences of nucleic acids. Their structure is formed by a chain of amino acids that form the main backbone to which nitrogenous bases are linked. These probes can be used in, but are not limited to, quantitative FISH or Q-FISH techniques.

Taqman probes specific for the genes SCIN, AKAP9, EDN-1, CXCL1, CXCL12 and CD70 are those listed in Table 1 of the examples of the present invention or other homologues.

A preferred embodiment of the kit for identifying senescent mesenchymal stem cells additionally comprises PNA probes, CEP probes and Taqman probes specific for the genes HIST1 H4C, HIST1 H4L, HIST1 H1C, CENPM, DYNLT3, SPC25, GTSE1, CDC45L, PLK1 and SKA3.

This kit can comprise, without any limitation, culture media, buffers, reagents and agents for the prevention of contamination. It can also additionally comprise primers, probes, fluorochromes, polymerases, antibodies, reagents, etc. The kit can also include methods and means that are necessary for carrying out the extraction, purification, etc. of nucleic acids and/or proteins. It may also include all the supports and recipients necessary for its operation and optimisation. Preferably, the kit additionally comprises the instructions to carry out the method of the invention. For illustrative purposes and without limiting the scope of the invention, the kit will contain the elements necessary for identifying senescent mesenchymal stem cells by the technique described above.

Throughout the description and the claims, the term "comprise" and its variants does not exclude other technical characteristics, additives, components or steps. For experts in the field, other purposes, advantages and characteristics of the invention will derive in part from the description and in part from the practice of the invention. The following examples and figures are provided for illustration purposes and are not intended to be limiting of the present invention.

DESCRIPTION OF THE FIGURES

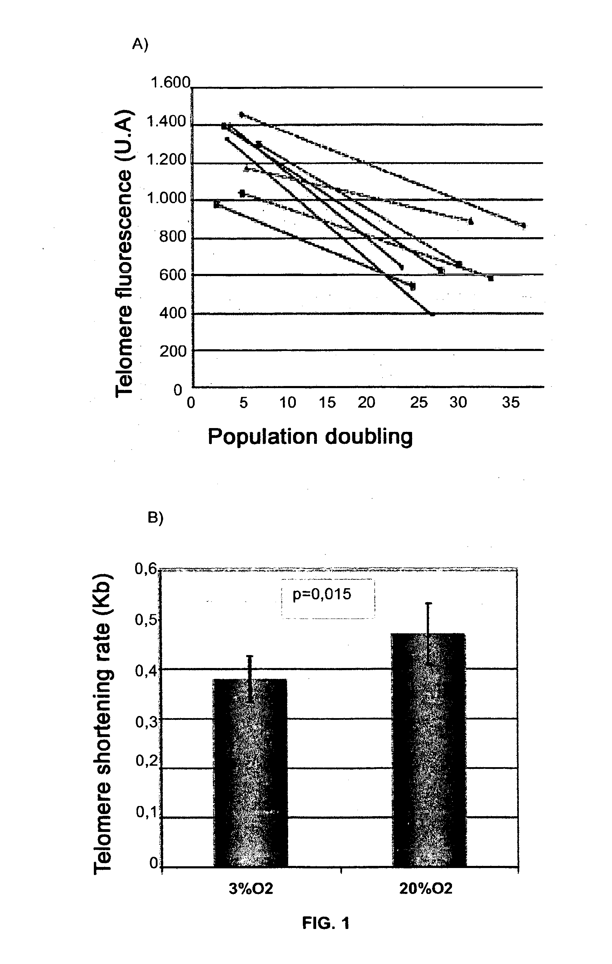

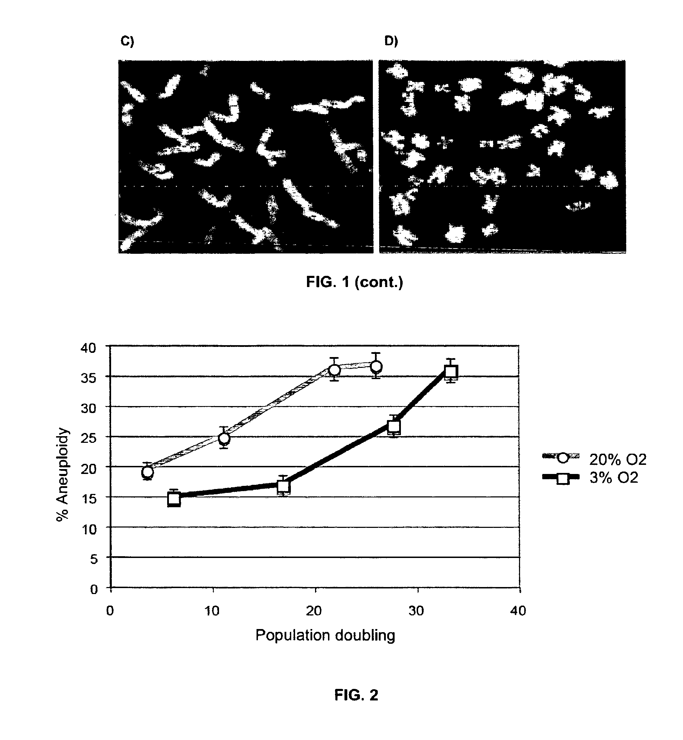

Fig. 1. A) Shows the analysis of the MSC telomere length by Q-FISH in MSCs in interphase of passages 2 and 15 against population doubling. The dotted lines represent shortening of the telomeres of MSCs growing in 3 % $O_2$. The continuous lines represent shortening of the telomeres of MSC growing in 20 % $O_2$. B) Shows the quantification of the loss of telomere length in Kb per population doubling. The conversion of telomere fluorescence to Kb was carried out using the fibroblastic lines TIN2 and TIN2-13. Loss of telomere length was 24 % less in 3 % $O_2$ than in 20 % $O_2$ (p=0.015). C-D) Shows examples of MSCs (C) in metaphase and hTert immortalised cells (D) hybridised with telomere PNA probes (bright signals at the ends of the chromosomes and counterstaining with DAPI).

Fig. 2. Shows the increase in aneuploidy levels with cell population doubling.

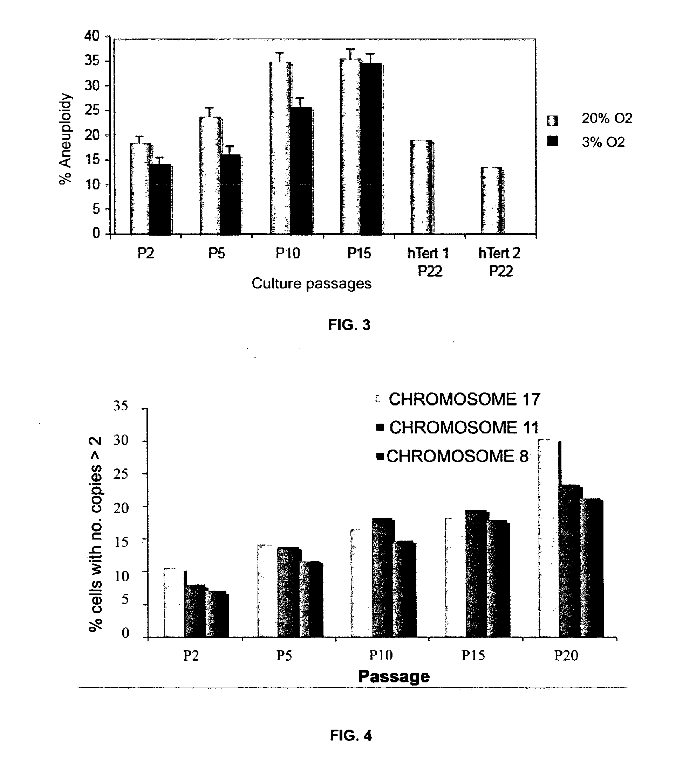

Fig. 3. Shows the aneuploidy levels of MSCs in various passages, under conditions of 20 % and 3 % $O_2$, and two independent lines of hTert at passage 22 (P22) under conditions of 20 % $O_2$. Determination by FISH with centromere probes for chromosomes 8, 13 and 17. Non-senescent cells (3 % $O_2$) have significantly reduced aneuploidy levels (p<0.05) from passage 2 onwards.

Fig. 4. Shows the percentage of aneuploid cells showing one or more extra copies of chromosomes 8, 11 and 17 at different culture passages. None of the chromosomes shows significant differences in the percentage of aneuploidy compared to the other chromosomes in any of the passages (P2-P20).

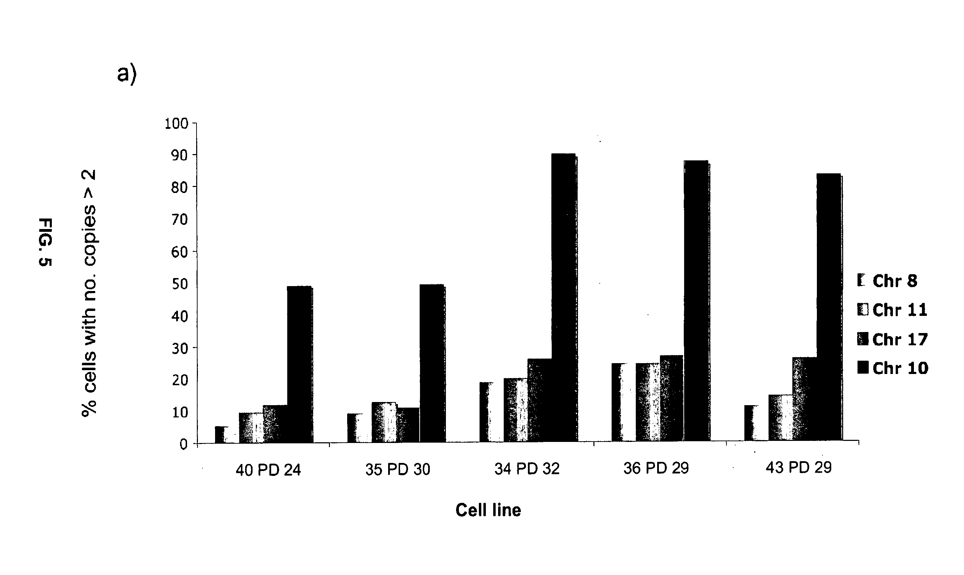

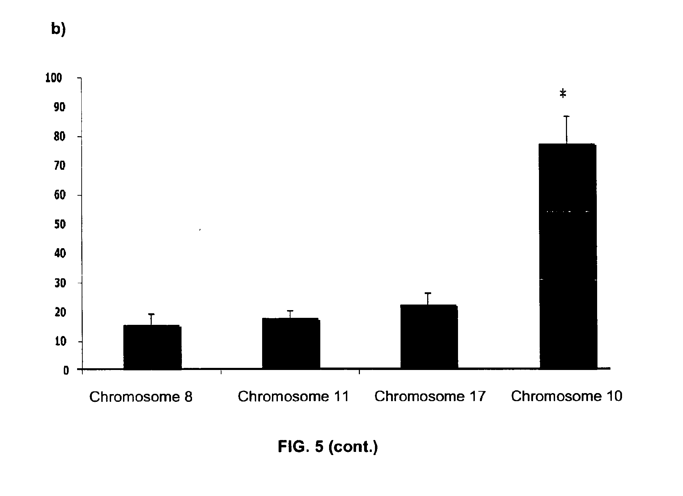

Fig. 5. a) Shows the percentage of aneuploid cells with a number of copies greater than 2 for chromosomes 8, 10, 11 and 17 at late passages (P20) of 4 independent lines of hMSCs. b) Shows the average percentage of aneuploid cells with a number of copies greater than 2 of the 4 lines of hMSCs at passage 20. Approximately 80 % of the hMSCs at the late passage maintained an extra copy of chromosome 10 (*= p<0.002) and only 20 % of the hMSCs at the late passage showed extra copies of the other chromosomes.

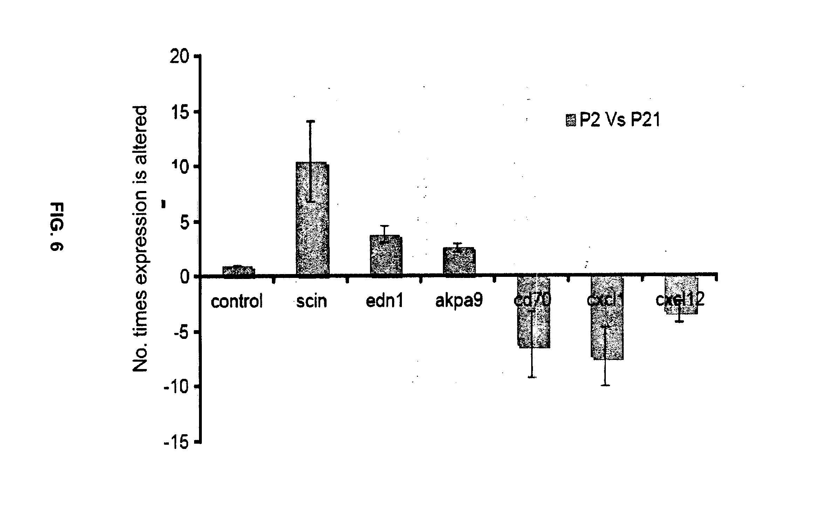

Fig. 6. Shows the quantification of mRNA transcripts of scinderin (scin), endothelin-1 (edn1), A-kinase anchor protein 9 (AKP9; Yotiao), cd70, chemokine (C-X-C) ligand 1 (Gro-alpha) and chemokine ligand 12 (stromal cell-derived factor-1) by RT-PCR using a Taqman assay in MSCs in passage 2 (P2) compared to passage 21 (P21). The bars represent the number of times that these genes are changed in four independent cell lines.

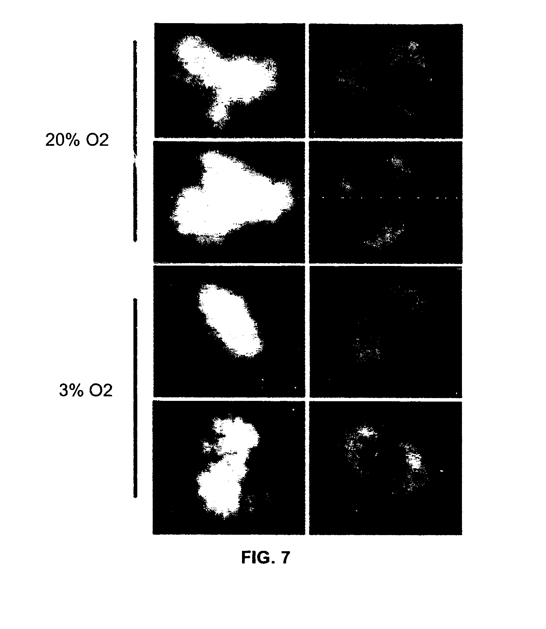

Fig. 7. Shows multipolar anaphases in MSCs growing in 20 % $O_2$. Aberrant mitosis spindles are observed with multiple poles, which can be seen owing to the staining with alpha-tubulin (panels to the right), and with abnormal chromosome alignments (panels to the left), staining with DAPI in the same cells. Examples of cells in normal anaphase with two normal mitosis poles in MSCs growing in 3 % $O_2$.

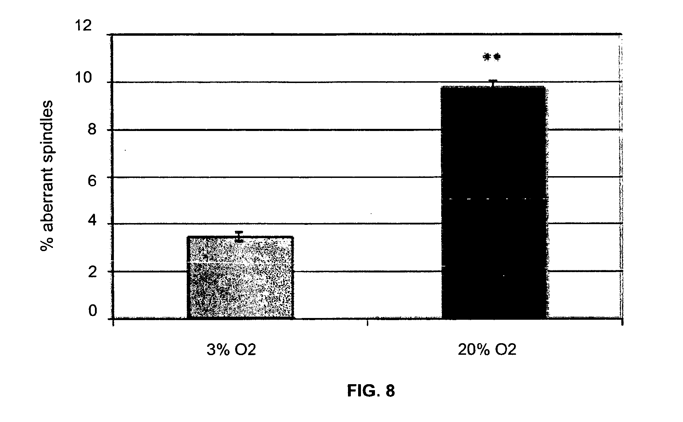

Fig. 8. Shows the quantification of the percentage of aberrant spindles in 40-50 cells analysed per cell line (n=4) and concentration of $O_2$ of the MSCs cultures in passage 20.

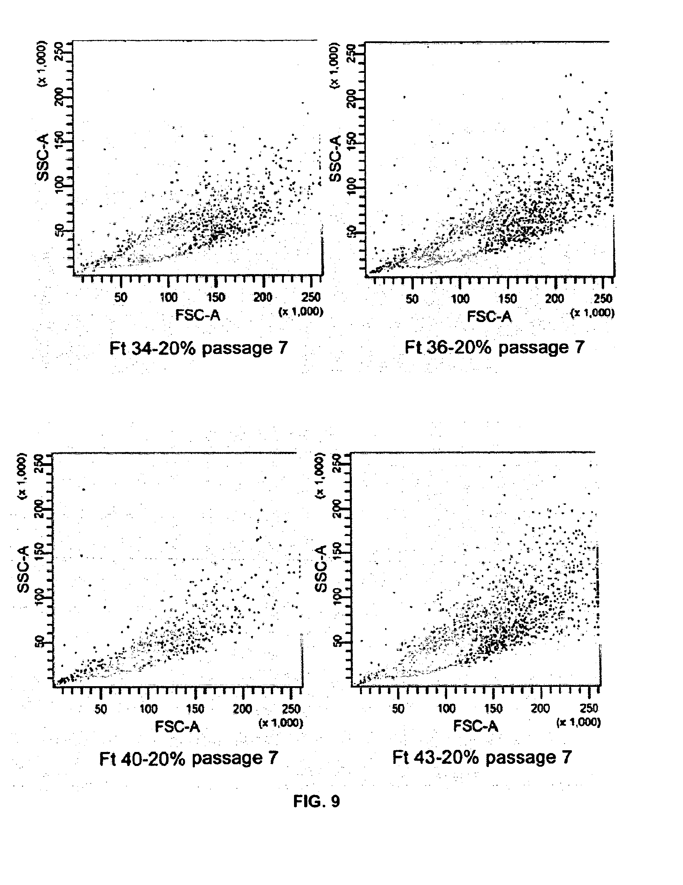

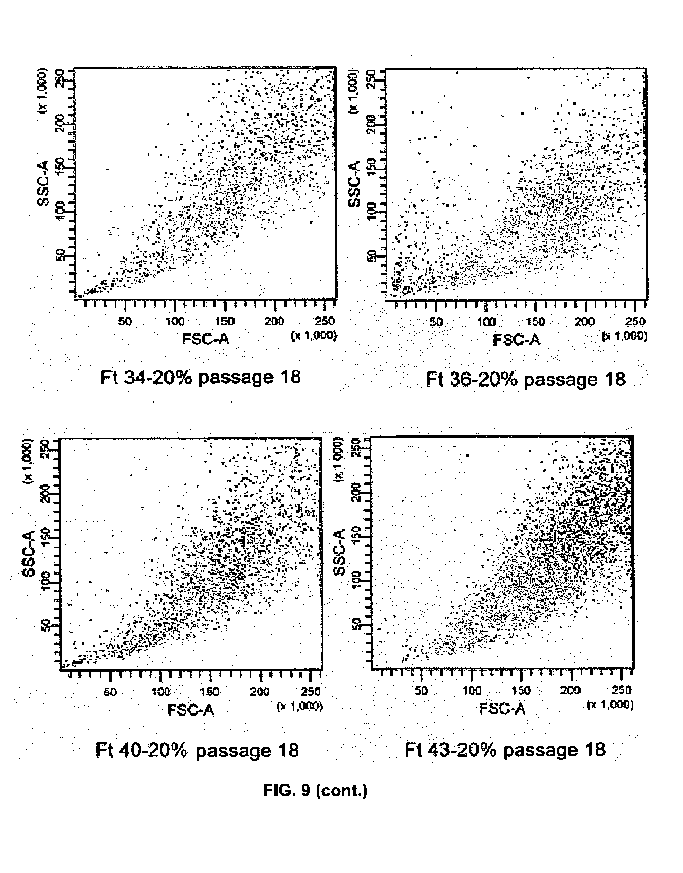

Fig. 9. Shows the results of flow cytometry.

Shows the size (FSC) and complexity (SSC) parameters for 4 independent lines of hMSCs in an early passage (above) and a late passage (below), showing an increase in cell size and complexity with senescence.

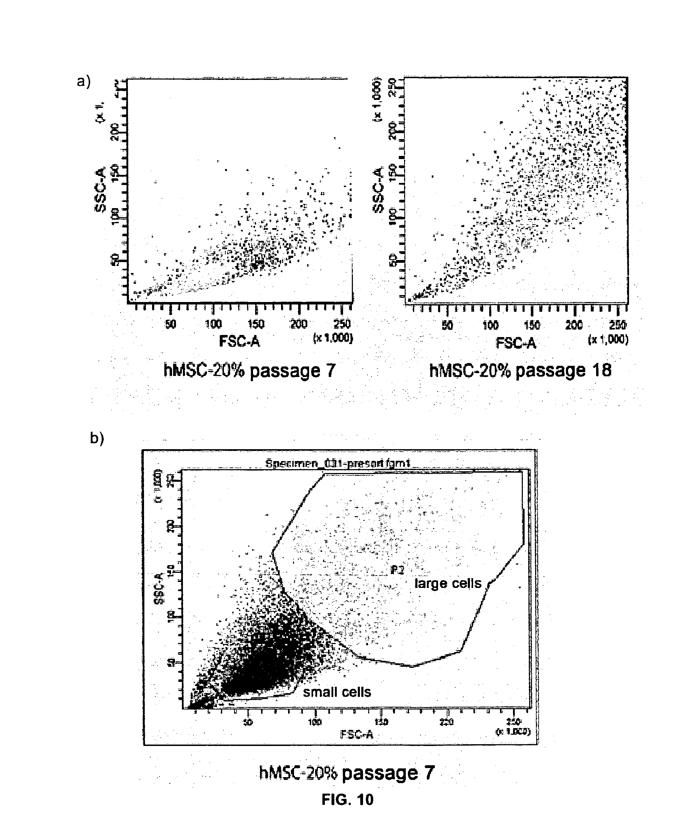

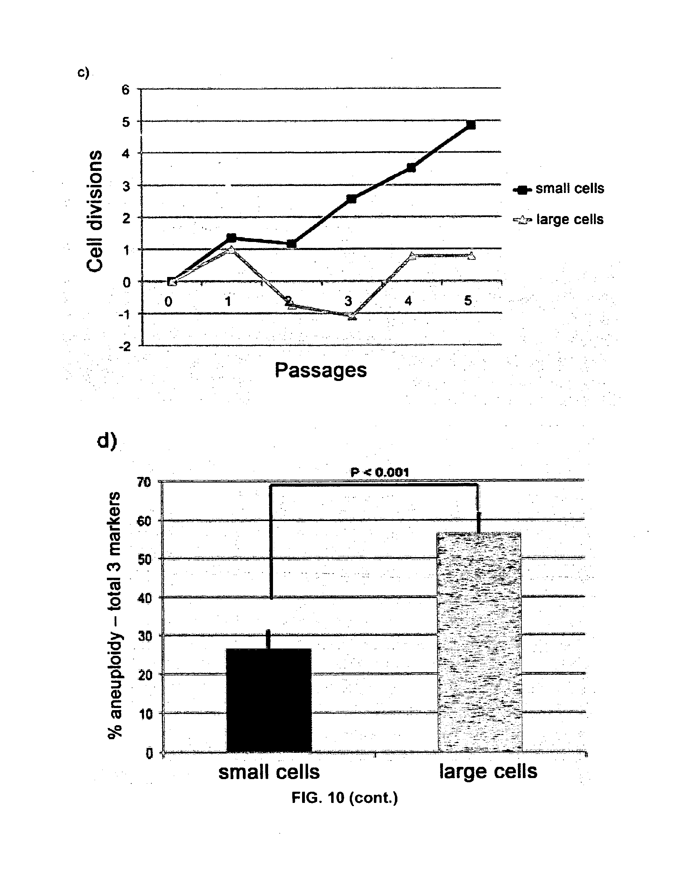

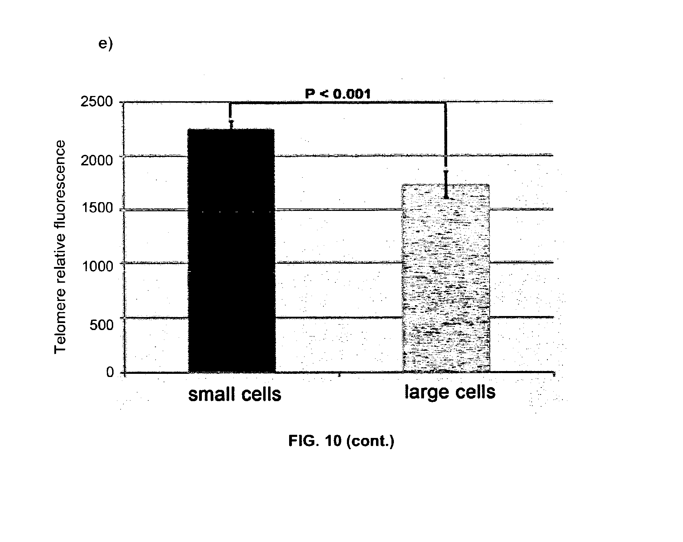

Fig. 10. Shows changes in cell size, senescence and the increase in genetic instability.

a) Example of flow cytometry showing the size (FSC) and complexity (SSC) parameters of a line of hMSC at an early passage (p7) and a late passage (p18). b) Selection of hMSC by flow cytometry at an average passage, selecting large and complex cells against small and less complex cells. c) Growth curve of the two cell sub-populations showing that large cells do not grow in culture and tend to be more senescent than their small homologues. d) Percentage of aneuploid cells for any of the 3 chromosomes 8, 11 and 17; almost 60 % of the large cells and only 25 % of the small cells are aneuploid. e) Relative quantification of telomere length of the two sub-populations showing that large cells have shorter telomeres confirming the hypothesis that these cells are more senescent.

EXAMPLES

The invention is illustrated below with tests carried out by the inventors demonstrating the effectiveness of the method for identifying senescent mesenchymal stem cells. These specific examples serve to illustrate the nature of the present invention and are only included for illustrative purposes and must not be interpreted as limitations of the invention claimed here. Therefore, the examples described below illustrate the invention without limiting its field of application.

EXAMPLE 1. Cells and culture conditions.

Human mesenchymal stem cells were obtained from the Inbiobank Stem Cell Bank (www.inbiobank.org) and came from adipose tissue. These cells exhibited the phenotype CD29+, CD73+ (SH3 and SH4), CD105+ (SH2), CD166+, CD45- and CD31-. All the cell donors were tested for HIV-1, HIV-2, hepatitis B and C and mycoplasma. The cell extraction process was carried out using liposuction and the cells were later treated with collagenase.

MSCs ($1-2 \times 10^4$ cells/ml) were cultured in a glucose-rich medium, Dulbecco's Modified Eagle's Medium (DMEM, Sigma, USA), supplemented with 10 % foetal bovine serum (FBS, Sigma, USA), glutamine and penicillin/streptomycin. The cells were cultured in the presence of two different $O_2$ concentrations, 20 % and 3 %, to obtain a senescent cell population and a non-senescent population respectively that could be compared. The cells capable of adhering to the plate were considered the first hMSC extract derived from adipose tissue. The medium was changed twice a week and the cells were passaged once every seven days until they reached an approximate confluence of 80 %. Cell growth *in vitro* was monitored and the cell number was counted with a haemocytometer.

EXAMPLE 2. Measurement of telomere shortening in human mesenchymal stem cells growing *in vitro*.

2.1. TELOMERE LENGTH ASSAY

To determine if senescence in human mesenchymal stem cells is due to telomere shortening, a quantitative fluorescence *in situ* hybridisation (Q-FISH) was carried out using a telomere PNA (Peptide Nucleic Acid) LL(CCCTAA) 3 probe labelled with Cy3 (Eurogentec) in the nucleus of MSCs in four different lines in interphase, growing in 20 % $O_2$ and in 3 % $O_2$, in passage 2 and in passage 15 (see Fig. 1 A). After hybridisation, the cells were washed three times in 0.1 % PBS Tween for 10 minutes at 60 °C and were dehydrated in ethanol for 5 minutes. Finally, they were contrast stained in Vectashield mountain medium with DAPI (Vector Laboratories Inc.). The images were taken with CytoVision. The telomere signals were captured at the same exposure time in all samples. Telomere length in Kb was calculated as a function of the fluorescence of 82-6 fibroblasts immortalised with hTert expressing the proteins TIN2 and TIN2-13 with stable and known telomere lengths (3.4 and 8.4 Kb respectively). The telomere signals of at least 20-30 nuclei per group were quantified using TFL-Telo version 2.

In cells growing in 20 % $O_2$, the average telomere length in passage 2 and in passage 15 were $8.43 \pm 1.28$ Kb and $3.76 \pm 0.7$ Kb respectively. The same cells in 3 % $O_2$ in the same passage numbers showed an average telomere length of $8.21 \pm 1.1$ Kb and $5.04 \pm 0.95$ Kb respectively, indicating that the cells growing in conditions of oxidative stress (20 % $O_2$) exhibited much greater shortening of DNA telomeres than cell cultured in 3 % $O_2$ (see Fig. 1 B). This demonstrates that the shortening ratio of the telomeres was $378 \pm 46$ and $470 \pm 61$ base pairs per cell division in 3 % $O_2$ and in 20 % $O_2$ respectively. This experiment suggests that growth of the MSCs depends on maintenance of telomeres.

2.2. TELOMERASE ACTIVITY TEST

As telomere length is strongly influenced by telomerase, the levels of activity of this enzyme were analysed using a TRAP assay based on Q-PCR. The telomerase assay was carried out in mesenchymal stem cells and in fibroblasts, which are considered to be telomerase negative. 5,000 MSCs were used per assay: 2 $\mu$l of extension reagent were added to 23 $\mu$l PCR reagent mixture containing (1 x PCR Master Mix power SYBR Green, 5mM EGTA, 4ng/$\mu$l Oligo ACX, 2ng/$\mu$l Oligo TS). PCR was carried out in 40 cycles at 94 °C for 10 minutes, at 94 °C for 15 seconds and at 60 °C for 1 minute. PCR continued with the ABI PRISM 7700 (Applied Biosystems) sequence detection system and the analysis was carried out using the 7900HT v2.3 (Applied Biosystems) software.

It was observed that primary mesenchymal stem cells had a level of telomerase activity equivalent to that of primary fibroblasts that are normally considered to be telomerase negative. In both cases, however, low levels of telomerase activity were detected owing to the sensitivity of the method.

To confirm that senescence of the MSCs depends on telomerase activity and on telomere length, the primary MSCs of passage 5 were incubated with a lentivirus (pRRL.SIN18) that codes for the catalytic subunit of the inverse transcriptase of human telomerase (hTert). To check the expression of telomerase after the infection, TRAP assays were carried out.

It was observed that the MSCs infected with lenti-hTert were immortalised, free of senescence and elongated their telomeres by two or three fold (see Fig. 1C-D).

These results indicate that senescence of human MSCs derived from adipose tissue is dependent on shortening of the telomeres and that infection of the catalytic subunit of human telomerase is sufficient to immortalise this cell type.

EXAMPLE 3. Analysis of ploidy level of human mesenchymal stem cells.

The ploidy levels in MSCs in interphase under both culture conditions with different oxygen concentrations were determined in passages 2 and 15 by FISH with centromere probes for chromosomes 8, 11 and 17 (see Fig. 2 and 3). Cells were incubated with 10 $\mu$g/ml Colcemid for 4 hours at 37 °C, then treated with 0.56 % KCI for 15 minutes at 37 °C and fixed in methanol:acetic acid (3:1) three times. The cell suspensions were deposited on slides and dried in air for 24 hours. Before hybridisation, the slides were treated with protease solution (0.1 % pepsin and HCI) and fixed in 4 % formaldehyde for 5 minutes at room temperature; next they were dehydrated by serial incubations in increasing ethanol concentrations (70, 90 and 100 %) for 5 minutes each. The cells were denatured in 70 % deionised formamide and 2X SSC for 5 minutes at 73 °C. Next they were dehydrated as described above. After dehydration, the cells were incubated for 24 h at 37 °C in darkness with 2-3 $\mu$l of commercial probes specific for the CEP and LS1 regions of the centromeres of chromosomes 8, 10, 11 and 17 (Breast Aneusomy Multi-Color Probe kit, http://www.abbottmolecular.com). The cells were washed in two passes with 0.4XSSC/0.3 % NP40 for 2 minutes at 73 °C and 2XSSC/0.1 % NP40 for 1 minute at room temperature; after washing, the chromosomes were stained using Vectashield mounting medium (VECTOR Laboratories) with DAPI (4,6-diamidine-2-phenylindole). Between 100 and 200 nuclei per cell line and passage were captured and the numbers of copies of each of the chromosomes were counted for each nucleus. The data were statistically analysed by the Student's t-test using 5 cell lines. The signals were taken with a Nikon 90i microscope with a plan fluor 100X objective lens [1,3 N/A] and suitable filters. The digital images were taken by Genus (CytoVision) software.

After investigating for the presence of chromosomal aneuploidy in interphase in 4 independent hMSC lines using probes specific for these three different chromosomes (8, 11 and 17), it was found that during prolonged cell culture there was a progressive increase in the aneuploidy levels for these three chromosomes (preferably towards an increase in the number of copies), without showing significant differences in the percentage of aneuploidy between chromosomes (see Fig. 4). It was observed that even in early passages, the aneuploidy levels for each of these chromosomes was 15 %. The results indicated that there is a tendency to increase aneuploidy with increasing culture passages under both concentrations of $O_2$, reaching 35 % in passage 15 (see Fig. 3).

Levels of chromosomal aneuploidy were also assessed for other chromosomes in cells that were grown until they became senescent. Thus, a high percentage of cells in interphase in passage 20 were found to show extra copies of chromosome 10 compared to the other chromosomes (see Fig. 5 a), reaching levels close to 80 % of cells with extra copies of chromosome 10 compared to percentages of around 20 % for the other 3 chromosomes (p<0.002) (see Fig. 5 b). These cytogenetic observations indicate: 1) that extra copies of chromosome 10 are associated with senescence of hMSCs and 2) that the acquisition of extra copies of chromosome 10 is giving these cells a selective advantage in culture conditions. Therefore, it is proposed that chromosome 10 can be used as a biomarker of senescence and genetic instability of hMSCs.

A possible inducing factor of aneuploidy is shortening of the telomeres. In order to determine if this shortening is responsible for the observed aneuploidy in MSCs, aneuploidy levels were measured in immortalised hTert-MSCs growing in 20 % $O_2$. These cells in passage 22 showed 19.48 % and 13.70 % aneuploidy (see Fig. 3), which is similar to the aneuploidy level of passage 2 of MSCs indicating that shortening of telomeres is an important factor that induces aneuploidy.

EXAMPLE 4. Profile of genetic expression of late passages of human mesenchymal stem cells: deregulation of the genes controlling the level of ploidy.

To search for possible biomarkers of genetic instability and uncover mechanisms that play an important role in senescence of human MSCs, an analysis of genetic expression of the MSCs growing in passage 2 (n=4) and in passage 21 (n=4) in 20 % $O_2$ was carried out. The protocol for the analysis of genetic expression based on single colour microarrays (Agilent Technologies, Palo Alto, CA, USA) was used to amplify and label the RNA. A retrotranscription of 400 ng of the total RNA was carried out via the primer T7 promoter and MMLV-RT. The cDNA was converted to aRNA using T7 RNA polymerase, which amplifies the target material and simultaneously incorporates CTP labelled with cyanine 3. The samples were hybridised to the microarray of the whole human genome 4 x 44 K (G4112F, Agilent Technologies). 1.65 $\mu$g of the aRNA labelled with Cy3 for 17 hours at 65 °C was hybridised in a hybridisation set in an oven (G2545A, Agilent) at 10 rpm in a final concentration of 1X GEx Hybridisation buffer HI-RPM. The arrays were washed and dried by centrifugation. They were scanned at a resolution of 5 mm in a DNA microarrays alignment scanner (G2565BA, Agilent Technologies). The images provided by the scanner were analysed using feature extraction software (Feature Extraction, Agilent Technologies).

The data from feature extraction were imported to the GeneSpring® GX version 9.0 software. (Agilent Technologies). The normalisation values and those of expression (log2 transformed) were quantified for each probe. The probes were also labelled (present, marginal, absent) by GeneSpring®. Those probes where the signal values were above the lowest percentile (20) and marked as present or marginal in 100 % of the replications in at least one of the two study conditions (23,716 probes) were selected for subsequent analysis.

Statistical analysis of the differential genetic expression between passage 21 and passage 2 was carried out by a two-class paired SAM test (Tusher, et al., 2001. Proc Natl Acad Sci USA, Vol. 98, 5116-5121).

An analysis of genetic enrichments and signalling pathways was performed using the *Ingenuity Pathway Analysis* (Ingenuity Systems®) software to generate an interaction network between the genes of interest and for functional analysis of the specific genes, and thereby to analyse what processes or signalling pathways are involved in genetic instability.

The RNA of the MSCs was extracted using the standard TRIzol method and the retrotranscription reaction was carried out using the SuperScript III (Invitrogen) inverse transcriptase kit. cDNA (10 ng) were added for reaction to 10 $\mu$l of 2x PCR Master Mix (Applied Biosystems). The Taqman probes that were used in the genetic expression assays for the quantification of mRNA are those described below in Table 1 (Applied Biosystems):

Table 1. Taqman probes used for the quantification of mRNA of the genes used as senescence biomarkers.

| | |
|---|---|
| CXCL1 | Hs00605382_gH |
| SCIN | Hs00263961_m1 |
| CXCL12 | Hs00171022_m1 |
| EDN1 | Hs01115919_m1 |
| AKAP9 | Hs00323978_m1 |
| CD70 | Hs00174297_m1 |

The PCR was loaded in an ABI PRISM 7700 apparatus and was quantified using the 7900HT v 2.3 software (Applied Biosystems).

These analyses indicated that the cells from later passages of cultivation overexpressed 23 genes and underexpressed 17 genes, q (%)<15 %. Functional analysis showed that there were 69 genes associated to cancer processes and the cell cycle that were significantly altered (p$\leq$ 0.02) in the gene expression data. In the cell cycle and cancer category, 8 genes were found to be significantly altered (FDR<12 %) (Table 2). Of these, 5 coded for proteins that are involved in ploidy and polyploidization: scinderin (SCIN), A-Kinase anchor protein 9 (AKAP9; Yotiao) and endothelin-1 (EDN-1), which were overexpressed 12.31, 2.36 and 1.89 times respectively, and CXCL12 and CXCL1 that were underexpressed 7.14 and 11 times respectively (Table 2). To verify the analysis of genetic expression and the analysis of signalling pathways, a RT-PCT was carried out for the expression of the genes SCIN, AKAP9, EDN1, CXCL1, CXCL12 and CD70 in MSCs in passage 2 and in passage 21 and a significant increase was observed in the expression of SCIN, AKAP9, EDN1 (p<0.05), a significant reduction in the expression of CXCL1 and CD70 (p<0.05) and a slight reduction in the expression of CXCL12 (p=0.09) (see Fig. 6).

Table 2. Results of the analysis of genetic expression of MSCs in passage 21 compared to the cells in passage 2. Only those genes identified by Ingenuity Pathway Analysis as expressed significantly differently (p<0.02) in the two groups and are involved in the cell cycle and ploidy control, are shown.

| Gene | Description | No. times altered | Log of no. times altered | q-value ( %) |
|---|---|---|---|---|
| SCIN | Scinderin (SCIN) | 12.31 | 3.62 | 2.95 |
| ACVR1C | Activin A receptor, type IC | 2.94 | 1.56 | 2.95 |
| AKAP9 | Kinase-A anchor protein 9 (Yotiao) | 2.36 | 1.24 | 11.50 |
| EDN1 | Endothelin 1 (EDN1) | 1.89 | 0.92 | 5.03 |
| CXCL2 | Chemokine (C-X-C) ligand 2 | 0.19 | -2.39 | 4.61 |
| CXCL12 | mRNA of protein FLJ00404 | 0.14 | -2.86 | 3.69 |
| CD70 | Molecule CD70 (CD70) | 0.10 | -3.36 | 3.69 |
| CXCL1 | Chemokine (C-X-C) ligand 1 | 0.09 | -3.45 | 0.00 |

As the FISH results showed that the MSCs growing in 20 % $O_2$ had a significant increase in aneuploidy compared with the same cells growing in 3 % $O_2$, we investigated whether some of the genes identified by gene expression analysis and signalling pathways analysis changed their expression with the concentration of $O_2$. Thus, in cells growing in the presence of 20 % $O_2$ there is an increase in the expression of SCIN, EDN-1 and AKAP9 and a reduction in the expression of CXCL12. This differential expression demonstrates the potential for these genes to be used as biomarkers of genetic stability and senescence in MSCs.

EXAMPLE 5. Analysis of the effects of *in vitro* expansion on the mitotic spindle of human mesenchymal stem cells growing in 20 % $O_2$.

The results of gene expression suggest that in addition to the shortening of the telomeres, defects in the mitotic spindle may be involved in the generation of aneuploidy in MSCs. In the light of this possibility, the percentage of poles in the mitotic spindles of MSCs was determined. MSCs growing in 20 % $O_2$ and 3 % $O_2$ in passages 18 to 20 were seeded on glass coverslips and treated with 0.1 % gelatin. The coverslips were washed twice in PBS, the cells fixed in 4 % paraformaldehyde in PBS for 15 minutes, permeabilised in 0.25 % Triton X-100 in PBS for 10 minutes and blocked in 0.1 % Tween, 1 % BSA in PBS for 1 hour. The coverslips were incubated with anti-alpha tubulin antibody (CP06 Calbiochem) (1:100) for 1 hour. Detection was carried out with a 1:200 dilution of goat anti mouse Alexa Fluor-488 conjugate as the secondary antibody (see Fig. 7). 32-49 mitotic cells per cell line in 4 independent isolates at each oxygen concentration were analysed. All the cell lines were analysed in passage 20 $\pm$ 2.

This analysis indicated that the MSCs growing in 20 % $O_2$ for 18-20 passages showed an average multipolar mitosis of 9.7 $\pm$ 0.29 %, whereas the same cells growing in 3 % $O_2$ only showed 3.4 $\pm$ 0.18 % defects per spindle (see Fig. 8). This indicates that senescent cells show a higher number of multipolar mitoses than non-senescent cells.

EXAMPLE 6. New statistical analysis of the expression array.

Labelling of the samples and microarray hybridisation, as well as data extraction, was carried out as described in Example 4. The data obtained were edited in R.

6.1. LABELLING OF SAMPLES AND MICROARRAY HYBRIDISATION

The one-colour microarray based on the protocol for the analysis of gene expression by Agilent Technologies (Palo Alto, CA, USA) was used to amplify and label the RNA. In summary, 400 ng of total RNA was retro-transcribed using primer T7 promoter and MMLV-RT. The cDNA was then converted to aRNA using T7 RNA polymerase, which simultaneously amplifies the target material and incorporates cyanine 3 labelled CTP. The RNA labelled with Cy3 (1.65 μg) was hybridised to a microarray of the full human genome 4 x 44K (G4112F, Agilent Technologies) for 17 hours at 65 °C in hybridisation buffer HI-RPM 1X GEx in a hybridisation oven (G2545A, Agilent Technologies) at 10 rpm. The arrays were washed according to the instructions, dried, centrifuged and scanned at 5 mm resolution in a DNA microarray scanner (G2565BA, Agilent Technologies) with corrections for defects in one-colour 4x44K microarray format. Scanning of the images was analysed using suitable software (Agilent Technologies).

## 6.2. DATA PROCESSING

The data were read in R and processed using the Bioconductor Agi4x44PreProcess package as follows: The options of the Agi4x44PreProcess were lists with the MeanSignal and the BGMedianSignal as foreground and background signals respectively. Next, the data were corrected and normalised according to the array background using the mean and the quantile methods. The method of the mean produced a positive background with a signal corrected by subtraction of the background signal from the foreground maintenance signal of any intensity less than or equal to 0.5 to produce corrected positive intensities. Then, the data were normalised between the arrays using the method of the quantile (Bolstad et al., 2003. Bioinformatics. Vol. 19, 185-193). A constant of 50 was added to the intensity before logarithmic transformation with the intention of reducing signal variability of low gene expression intensities. The AFE image analysis software attaches to each characteristic a set of indicators identifying different signal quantification properties. Agi4x44PreProcess uses these indicators to filter the characteristics that are 1) controls, 2) are outside the dynamic range of the scanner and 3) are atypical values. To maintain the characteristics within the dynamic range, 3 independent levels of filtration can be applied, so ensuring that 1) the signal is distinguished from the background, 2) the signal is found and 3) the signal is not saturated.

For each of these filtration passes, it was necessary that each characteristic had at least 75 % of its replicates within one experimental condition and that it had a signal quantification that indicated that the signal was within the dynamic range. In addition, for each characteristic of replicates over the whole set of samples, those probes that had more than 25 % of these replicates in at least one experimental condition with one signal indicating the presence of atypical values were filtered. After the finalisation of all the preprocessing steps, 26,670 (p21 against p2) characteristics were available for statistical analysis. Finally, Agi4x44PreProcess mapped each corresponding Agilent probe to the access number, gene symbol, gene description and gene ontology identifiers (GO) (Genetic Ontology Consortium) using the annotation provided by Bioconductor *hgug4112a.db.*

## 6.3. STATISTICAL ANALYSIS

Analysis of differential expression was carried out using the characteristics of linear modelling employed in the Bioconductor limma package. The limma package incorporates empirical Bayes methods (Smyth, 2004. Stat Appl Genet Mol Biol. Vol. 3, Article 3) to obtain moderated statistics. To estimate the differential expression between the different experimental conditions in the data set (P21 vs. P2), the following linear modelling was applied to each gene:

$$y_{ij} = \tau_i + e_{ij}$$

where $y_{ij}$ is the observation of the ith treatment for the jth individuals, $\tau_i$ is the effect of the ith treatment and $e_{ij}$ is the experimental error, assuming a normal distribution with average 0 and variance $\sigma_e^2$. The genes that are differentially expressed due to differences in the treatments are determined by comparing each gene of the hypothesis of no differences between signals under different treatments using the estimation, $\tau_i$.

To reduce the number of genes for the correction of multiple comparisons. Without loss of important information, a non-specific filtration was carried out discarding the genes that showed a constant expression between the samples (IQR<0.5) or low expression of the signal (log2 expression < 5 in all samples) using the function Bioconductor *genefilter.* Multiple comparison of genes was carried out taking into account the rate of false determinations, which was estimated by the value of the statistic q (Storey and Tibshirani, 2003. Proc Natl Acad Sci USA., Vol. 100, 9440-9445) using the Bioconductor qvalue.

To integrate the significant expression profiles into functional categories, gene ontology (GO) was carried out based on statistical analysis using the hyperGTest function of the GOstats package. HyperGTest calculates the values of p hypergeometries to compare over-representation and under-representation of each GO term in a given subset of genes against the distribution of GO terms defined in a gene universe. The universe includes those genes that are above background signal and have a known GO term in the corresponding database. Duplicated genes were removed before GO analysis.

6.4. RESULTS OBTAINED. SELECTED BIOMARKERS

6.4.1. Biomarkers related to genetic stability.

The genes selected for differential deregulation are genes coding for histones and for the HMGA1 protein. The following table shows that there is a clear pattern in the increase in the expression of all the histones in the 2HB family and a reduction in the expression of histones in the H4 family. The other histones showed changes by no clear pattern.

Table 3. Gene set for Histones and HGMA1.

| SYMBOL | NAME | Change (Log2) | FDR BH (t) | FDR q value (t) |
| --- | --- | --- | --- | --- |
| HIST2H2AC | Histone group 2, H2ac | -0.744 | 0.06824 | 0.0346 |
| HIST1H4C | Histone group 1, H4c | -1.025 | 0.06824 | 0.0346 |
| HIST1H2BK | Histone group 1, H2bk | 0.994 | 0.06824 | 0.0346 |
| HIST1H4L | Histone group 1, H4l | -1.05 | 0.06824 | 0.0346 |
| HIST1H2BE | Histone group 1, H2be | 0.872 | 0.06824 | 0.0346 |
| HST1H4B | Histone group 1, H4b | -0.977 | 0.06824 | 0.0346 |
| HIST1H2BO | Histone group 1, H2bo | 0.963 | 0.06824 | 0.0346 |
| HIST1H4D | Histone group 1, H4d | -0.999 | 0.0684 | 0.03468 |
| HIST1H2BL | Histone group 1, H2bl | 0.897 | 0.0684 | 0.03468 |
| HIST1H2BF | Histone group 1, H2bf | 0.882 | 0.0684 | 0.03468 |
| HIST1H2BH | Histone group 1, H2bh | 0.91 | 0.07012 | 0.03555 |
| HIST1H3D | Histone group 1, H3d | 0.785 | 0.0728 | 0.03691 |
| HIST1H1A | Histone group 1, H1a | -0.876 | 0.07288 | 0.03695 |
| HIST1H2BI | Histone group 1, H2bi | 0.79 | 0.07288 | 0.03695 |
| HIST1H1B | Histone group 1, H1b | -0.623 | 0.07445 | 0.03775 |
| HIST1H2BB | Histone group 1, H2bb | 0.816 | 0.07445 | 0.03775 |
| HIST1H2AE | Histone group 1, H2ae | 0.84 | 0.07445 | 0.03775 |
| HMGA1 | High mobility group AT-hook 1 | -0.738 | 0.07744 | 0.03926 |
| HIST1H2BC | Histone group 1, H2bc | 0.833 | 0.07744 | 0.03926 |
| HIST1H1C | Histone group 1, H1c | 1.433 | 0.07744 | 0.03926 |
| HIST1H2BD | Histone group 1, H2bd | 0.721 | 0.08786 | 0.04455 |
| HIST1H2BM | Histone group 1, H2bm | 0.666 | 0.08786 | 0.04455 |
| HIST1H2BJ | Histone group 1, H2bj | 0.784 | 0.08786 | 0.04455 |
| HIST1H2BN | Histone group 1, H2bn | 0.747 | 0.08886 | 0.04506 |
| HIST2H2AA4 | Histone group 2, H2aa4 | 0.947 | 0.09486 | 0.0481 |
| HIST2H2BE | Histone group 2, H2be | 0.692 | 0.09507 | 0.04821 |
| HIST1H2AD | Histone group 1, H2ad | 0.87 | 0.09507 | 0.04821 |

6.4.2. Biomarkers related to genetic stability and more specifically to chromosomal segregation during mitosis.

Chromosomal segregation during mitosis is one of the most important causes of genetic stability. Those markers that had a pValue ≤ 0.05 were selected. The marker genes are shown in the following table. It shows that expression of all the genes coding for the proteins of the complex associated to the centromere (CENP) was reduced except in one case.

Table 4. Genes of the centromere nucleosome associated complex (CENPA-NAC).

| SYMBOL | NAME | Change (Log2) | FDR BH (t) | FDR q value (t) |
|--------|------|---------------|------------|-----------------|
| CENPM | Protein of centromere M | -1.251 | 0.07744 | 0.03926 |
| CENPO | Protein of centromere O | -0.52 | 0.11434 | 0.05798 |
| CENPA | Protein of centromere A | -0.703 | 0.12857 | 0.06519 |
| CENPN | Protein of centromere N | -0.205 | 0.31661 | 0.16054 |
| CENPV | Protein of centromere V | -0.269 | 0.37008 | 0.18765 |
| CENPK | Protein of centromere K | -0.213 | 0.54428 | 0.27597 |
| CENPF | Protein of centromere F, 350/400ka (mitosin) | -0.51 | 0.61506 | 0.31186 |
| CENPQ | Protein of centromere Q | 0.079 | 0.76654 | 0.38867 |

6.4.3. Biomarkers related to centrosome, centromere and kinetochore stability (mitotic spindle).

The stability of the centrosome, centromere and kinetochore are subfunctions affecting chromosomal segregation. Table 6 shows that of all the markers selected (26), the majority (21) showed reduced expression.

Table 5. Genes of the mitotic spindle.

| SYMBOL | NAME | Change (Log2) | FDR BH (t) | FDR q value (t) |
|--------|------|---------------|------------|-----------------|
| DYNLT3 | Dynein light chain Tctex-type 3 | 1.07 | 0.06824 | 0.0346 |
| SPC25 | SPC25, NDC80 kinetochore complex component, homolog (*S. cerevisiae*) | -1.466 | 0.07207 | 0.03654 |
| TNFAIP3 | Tumour necrosis factor, inducing alpha-protein 3 | -0.988 | 0.07445 | 0.03775 |
| GTSE1 | G-2 and S-phase expressed 1 | -1.569 | 0.07677 | 0.03892 |
| HAUS8 | HAUS complexes similar to augmin, subunit 8 | -0.643 | 0.07677 | 0.03852 |
| CDC45L | CDC45 cell division cycle 45-like (S. *cerevisiae*) | -1.832 | 0.07677 | 0.03892 |
| SKA1 | Spindle and kinetochore associated complex subunit 1 | -0.79 | 0.07744 | 0.03926 |
| PLK1 | Polo-like kinase 1 *(Drosophila)* | -1.748 | 0.07744 | 0.03926 |
| sep-07 | Septin 7 | 0.848 | 0.08471 | 0.04295 |
| CEP63 | Centrosomal protein 63 kDa | 0.545 | 0.08786 | 0.04455 |
| SKA3 | Spindle and kinetochore associated complex subunit 3 | -1.263 | 0.09507 | 0.04821 |
| KIF22 | Kinesin family member 22 | -0.909 | 0.09507 | 0.04821 |
| ESPL1 | Extra body 1 homologue to the pole of the spindle (*S. cerevisiae*) | -0.935 | 0.09622 | 0.04879 |
| PXN | Paxilline | -0.793 | 0.09916 | 0.05028 |
| BIRC5 | Baculoviral IAP repeat-containing 5 | -1.415 | 0.10174 | 0.05159 |
| BUB1B | budding uninhibited by *benzimidazoles 1 homolog* | -1.17 | 0.10174 | 0.05159 |
| CEP290 | Centrosomal protein 290 kDa | 0.965 | 0.10174 | 0.05159 |
| CCNB1 | Cyclin B1 | -0.978 | 0.10196 | 0.0517 |
| KIFC1 | Kinesin family member C1 | -1.61 | 0.10218 | 0.05181 |

(continued)

| SYMBOL | NAME | Change (Log2) | FDR BH (t) | FDR q value (t) |
|--------|------|---------------|------------|------------------|
| SPAG5 | Antigen associated with sperm 5 | -1.418 | 0.10245 | 0.05195 |
| NDC80 | NDC80 homolog kinetochore complex component (*S. cerevisiae)* | -1.242 | 0.10412 | 0.05279 |
| HAUS7 | HAUS similar to augmin complex, subunit 7 | -0.562 | 0.1057 | 0.0536 |
| EVI5 | Site of ectopic viral integration 5 | 0.415 | 0.10859 | 0.05506 |
| KIF4A | Kinesin family member 4A | -1.089 | 0.10934 | 0.05544 |
| CDC25B | Cell cycle division homolog to 25 B (S. *pombe*) | -1.453 | 0.11521 | 0.05842 |
| KIF20A | Kinesin family member 20A | -1.18 | 0.11801 | 0.05984 |

6.5. CELL SIZE, SENESCENCE AND INCREASE IN GENETIC INSTABILITY

As is well known, cell size is related to cell ploidy (reviewed in Strochova and Pellman, 2004. Nat Rev Mol Cell. Biol., Vol. 5). Some authors have stated that each doubling of genetic material is accompanied by an increase of cell volume of approximately 2 times in human and mouse hepatocytes. Similarly, the increase in cell size is one of the most classic characteristics shown by senescent cells. Based on these premises, we decided to select cells of larger size and complexity (cells that are accumulated during passages [Fig. 9]) at a specific passage. We were able to show that these larger sized cells were incapable of replication during culture over time and they also exhibited smaller telomere length and a high accumulation of aneuploidy compared to controls (small and less complex cells) (Fig. 10). These observations indicated that increase in aneuploidy in interphase is a strong biomarker of senescence of cells in culture.

**Claims**

1. Method for identifying senescent mesenchymal stem cells, comprising:

   a. measuring the length of chromosome telomeres of the mesenchymal stem cell,
   b. determining the ploidy level of the mesenchymal stem cell of step (a),
   c. analyzing the presence of multipolar mitosis in the mesenchymal stem cell of step (b),
   d. analyzing the level of expression of the genes SCIN, EDN-1, CXCL1, CXCL12 and CD70 in the mesenchymal stem cell of step (c), and
   e. associating the data obtained in steps (a)-(d) with a senescent phenotype.

2. Method for identifying senescent mesenchymal stem cells according to claim 1 wherein the measurement of the length of chromosome telomeres of step (a) is carried out by at least one of the following methods: quantitative FISH and/or TRAP.

3. Method for identifying senescent mesenchymal stem cells according to any one of claims 1 or 2 wherein the determination of the ploidy level of step (b) is carried out by hybridisation with specific chromosomal probes and subsequent counting of their fluorescent signal.

4. Method for identifying senescent mesenchymal stem cells according to claim 3 wherein the specific chromosomal probes are CEP probes.

5. Method for identifying senescent mesenchymal stem cells according to claim 4 wherein the CEP probes are specific for chromosomes 8, 10, 11 and/or 17.

6. Method for identifying senescent mesenchymal stem cells according to claim 5 wherein the CEP probes are specific for chromosome 10.

7. Method for identifying senescent mesenchymal stem cells according to any one of claims 1 to 6 wherein the senescent phenotype of step (e) is **characterised by** showing:

a. a reduction in chromosome telomere length compared to a non-senescent cell,
b. aneuploidy,
c. multipolar mitosis,
d. an increased expression of the genes SCIN and EDN-1, and
e. a reduced expression of the genes CXCL1, CXCL12 and/or CD70.

8.  Method for identifying senescent mesenchymal stem cells according to any one of claims 1 to 7 wherein the mesenchymal stem cells come from a human.

9.  Method for identifying senescent mesenchymal stem cells according to any one of claims 1 to 8, wherein additionally the levels of expression of the genes HIST1H4C, HIST1H4L, HIST1H1C, CENPM, DYNLT3, SPC25, GTSE1, CDC45L, PLK1 and SKA3 in the mesenchymal stem cell are analysed, and the data obtained are associated with the senescent phenotype.

10. Method for identifying senescent mesenchymal stem cells according to claim 9, wherein the senescent phenotype is **characterised by** showing:

    a. an increased expression of the genes HIST1H1C and DYNLT3, and
    b. a reduced expression of the genes HIST1 H4C, HIST1 H4L, CENPM, SPC25, GTSE1, CDC45L, PLK1 and SKA3.

11. Kit for identifying senescent mesenchymal stem cells comprising: PNA telomere probes, CEP probes and Taqman probes that are specific for the genes SCIN, EDN-1, CXCL1, CXCL12 and CD70.

12. Kit for identifying senescent mesenchymal stem cells according to claim 11 wherein the CEP probes are specific for chromosomes 8, 10, 11 and/or 17.

13. Kit for identifying senescent mesenchymal stem cells according to any one of claims 11 or 12, additionally comprising PNA probes, CEP probes and Taqman probes specific for the genes HIST1H4C, HIST1H4L, HIST1H1C, CENPM, DYNLT3, SPC25, GTSE1, CDC45L, PLK1 and SKA3.


**Patentansprüche**

1.  Verfahren zur Identifizierung von alternden mesenchymalen Stammzellen, bestehend aus:

    a. Messen der Länge der Chromosomentelomere der mesenchymalen Stammzelle,
    b. Bestimmen des Ploidiegrads der mesenchymalen Stammzelle aus Schritt (a),
    c. Analyse des Vorhandenseins einer multipolaren Mitose in der mesenchymalen Stammzelle aus Schritt (b),
    d. Analyse der Expressionsstufe der Gene SCIN, EDN-1, CXCL1, CXCL12 und CD70 der mesenchymalen Stammzelle aus Schritt (c), und
    e. Assoziation der bei den Schritten (a) bis (d) erhaltenen Daten mit einem alternden Phänotyp.

2.  Verfahren zur Identifizierung von alternden mesenchymalen Stammzellen nach Anspruch 1, wobei das Messen der Länge der Chromosomentelomere von Schritt (a) durch mindestens eine der folgenden Verfahren durchgeführt wird: Q-FISH und/oder TRAP.

3.  Verfahren zur Identifizierung von alternden mesenchymalen Stammzellen nach einem der Ansprüche 1 oder 2, wobei das Bestimmen des Ploidiegrads von Schritt (b) durch Hybridisierung mit spezifischen Chromosomenproben und einem anschließenden Zählen ihres fluoreszierenden Signals durchgeführt wird.

4.  Verfahren zur Identifizierung von alternden mesenchymalen Stammzellen nach Anspruch 3, wobei die spezifischen Chromosomenproben CEP-Proben sind.

5.  Verfahren zur Identifizierung von alternden mesenchymalen Stammzellen nach Anspruch 4, wobei die CEP-Proben spezifisch für die Chromosomen 8, 10, 11 und/oder 17 sind.

6.  Verfahren zur Identifizierung von alternden mesenchymalen Stammzellen nach Anspruch 5, wobei die CEP-Proben spezifisch für Chromosom 10 sind.

7. Verfahren zur Identifizierung von alternden mesenchymalen Stammzellen nach einem der Ansprüche 1 bis 6, wobei der alternde Phänotyp von Schritt (e) **dadurch gekennzeichnet ist, dass** er Folgendes aufweist:

   a. eine Reduktion der Länge des Chromosomentelomers, im Vergleich zu einer nicht-alternden Zelle
   b. Aneuploidie,
   c. multipolare Mitose,
   d. eine erhöhte Expression der Gene SCIN und EDN-1, und
   e. eine reduzierte Expression der Gene CXCL1, CXCL12 und/oder CD70.

8. Verfahren zur Identifizierung von alternden mesenchymalen Stammzellen nach einem der Ansprüche 1 bis 7, wobei die mesenchymalen Stammzellen von einem Menschen stammen.

9. Verfahren zur Identifizierung von alternden mesenchymalen Stammzellen nach einem der Ansprüche 1 bis 8, wobei zusätzlich die Expressionsstufe der Gene HIST1H4C, HIST1H4L, HIST1H1C, CENPM, DYNLT3, SPC25, GTSE1, CDC45L, PLK1 und SKA3 in der mesenchymalen Stammzelle analysiert und die erhaltenen Daten mit dem alternden Phänotyp assoziiert werden.

10. Verfahren zur Identifizierung von alternden mesenchymalen Stammzellen nach Anspruch 9, wobei der alternde Phänotyp **dadurch gekennzeichnet ist, dass** er Folgendes aufweist:

    a. eine erhöhte Expression der Gene HIST1H1C und DYNLT3, und
    b. eine reduzierte Expression der Gene HIST1 H4C, HIST1 H4L, CENPM, SPC25, GTSE1, CDC45L, PLK1 und SKA3.

11. Ausstattung zur Identifizierung von alternden mesenchymalen Stammzellen, bestehend aus: PNA-Proben, CEP-Proben und Taqman-Proben, die spezifisch für die Gene SCIN, Telomer, EDN-1, CXCL1, CXCL12 und CD70 sind.

12. Ausstattung zur Identifizierung von alternden mesenchymalen Stammzellen nach Anspruch 11, wobei die CEP-Proben spezifisch für die Chromosomen 8, 10, 11 und/oder 17 sind.

13. Ausstattung zur Identifizierung von alternden mesenchymalen Stammzellen nach einem der Ansprüche 11 bis 12, zusätzlich umfassend PNA-Proben, CEP-Proben und Taqman-Proben, die spezifisch für die Gene HIST1H4C, HIST1H4L, HIST1H1C, CENPM, DYNLT3, SPC25, GTSE1, CDC45L, PLK1 und SKA3 sind.


**Revendications**

1. Procédé pour identifier des cellules souches mésenchymateuses sénescentes, consistant à :

   a. mesurer la longueur des télomères de chromosome de la cellule souche mésenchymateuse,
   b. déterminer le degré de ploïdie de la cellule souche mésenchymateuse de l'étape (a),
   c. analyser la présence de mitose multipolaire dans la cellule souche mésenchymateuse de l'étape (b),
   d. analyser le niveau d'expression des gènes SCIN, EDN-1, CXCL1, CXCL12 et CD70 dans la cellule souche mésenchymateuse de l'étape (c), et
   e. associer les données obtenues dans les étapes (a)-(d) à un phénotype sénescent.

2. Procédé pour identifier des cellules souches mésenchymateuses sénescentes selon la revendication 1 dans lequel la mesure de la longueur des télomères de chromosome de l'étape (a) est réalisée selon l'un au moins des procédés suivants : FISH (hybridation in situ en fluorescence) quantitative et/ou TRAP (protocole d'amplification de répétition de télomère).

3. Procédé pour identifier des cellules souches mésenchymateuses sénescentes selon n'importe laquelle des revendications 1 ou 2, dans lequel la détermination du degré de ploïdie de l'étape (b) est réalisée par hybridation avec des sondes chromosomiques spécifiques et ensuite le comptage de leur signal de fluorescence.

4. Procédé pour identifier des cellules souches mésenchymateuses sénescentes selon la revendication 3, dans lequel les sondes chromosomiques spécifiques sont des sondes CEP.

5. Procédé pour identifier des cellules souches mésenchymateuses sénescentes selon la revendication 4, dans lequel les sondes CEP sont spécifiques aux chromosomes 8, 10, 11 et/ou 17.

6. Procédé pour identifier des cellules souches mésenchymateuses sénescentes selon la revendication 5, dans lequel les sondes CEP sont spécifiques au chromosome 10.

7. Procédé pour identifier des cellules souches mésenchymateuses sénescentes selon n'importe laquelle des revendications 1 à 6, dans lequel le phénotype sénescent de l'étape (e) est **caractérisé par le fait qu'**il montre :

   a. une réduction dans la longueur du télomère de chromosome par comparaison avec une cellule non sénescente,
   b. aneuploïdie
   c. mitose multipolaire,
   d. une expression accrue des gènes SCIN et EDN-1, et
   e. une expression réduite des gènes CXCL1, CXCL12 et/ou CD70.

8. Procédé pour identifier des cellules souches mésenchymateuses sénescentes selon n'importe laquelle des revendications 1 à 7, dans lequel les cellules souches mésenchymateuses proviennent d'un humain.

9. Procédé pour identifier des cellules souches mésenchymateuses sénescentes selon n'importe laquelle des revendications 1 à 8, dans lequel, en outre, les niveaux d'expression des gènes HIST1H4C, HIST1H4L, HIST1H1C, CENPM, DYNLT3, SPC25, GTSE1, CDC45L, PLK1 et SKA3 dans la cellule souche mésenchymateuse sont analysés, et les données obtenues sont associées au phénotype sénescent.

10. Procédé pour identifier des cellules souches mésenchymateuses sénescentes selon la revendication 9, dans lequel le phénotype sénescent est **caractérisé par le fait qu'**il montre :

   a. une expression accrue des gènes HIST1H1C et DYNLT3, et
   b. une expression réduite des gènes HIST1H4C, HIST1H4L, CENPM, SPC25, GTSE1, CDC45L, PLK1 et SKA3.

11. Kit pour identifier des cellules souches mésenchymateuses sénescentes comprenant : des sondes télomères PNA, des sondes CEP et des sondes Taqman qui sont spécifiques pour les gènes SCIN, EDN-1, CXCL1, CXCL12 et CD70.

12. Kit pour identifier des cellules souches mésenchymateuses sénescentes selon la revendication 11, dans lequel les sondes CEP sont spécifiques pour les chromosomes 8, 10, 11 et/ou 17.

13. Kit pour identifier des cellules souches mésenchymateuses sénescentes selon n'importe laquelle des revendications 11 ou 12, comprenant en outre des sondes PNA, des sondes CEP et des sondes Taqman spécifiques pour les gènes HIST1H4C, HIST1H4L, HIST1H1C, CENPM, DYNLT3, SPC25, GTSE1, CDC45L, PLK1 et SKA3.

A)

B)

FIG. 1

C)

D)

**FIG. 1 (cont.)**

**FIG. 2**

**FIG. 3**

**FIG. 4**

a)

FIG. 5

b)

FIG. 5 (cont.)

**FIG. 6**

20% O2

3% O2

**FIG. 7**

FIG. 8

Ft 34-20% passage 7

Ft 36-20% passage 7

Ft 40-20% passage 7

Ft 43-20% passage 7

FIG. 9

Ft 34-20% passage 18

Ft 36-20% passage 18

Ft 40-20% passage 18

Ft 43-20% passage 18

FIG. 9 (cont.)

a)

hMSC-20% passage 7          hMSC-20% passage 18

b)

hMSC-20% passage 7

FIG. 10

c)

d)

FIG. 10 (cont.)

e)

FIG. 10 (cont.)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 2002123526 A1

**Non-patent literature cited in the description**

• **KRZYSZTOF KSIAZEK.** *Rejuvenation Research,* 2009, vol. 12 (2), 105-116
• **REZA IZADPANAH et al.** *Cancer Research,* 2008, vol. 68 (11), 4229-4238
• **STEVEN R. SCHWARZE et al.** *Neoplasia,* 2005, vol. 7 (9), 816-823
• **SPITS, C. et al.** *Nature Biotechnology,* 2008, vol. 26, 1361-1363
• **MELISSA A. BAXTER et al.** *Stem Cells,* 2004, vol. 22, 675-682
• **EUNSOOK RYU et al.** *Biochemical and Biophysical Research Communications,* 2008, vol. 371, 431-436

• **WOLFGANG WAGNER et al.** *PLoS ONE,* 2008, vol. 3 (5
• **TUSHER et al.** *Proc Natl Acad Sci USA,* 2001, vol. 98, 5116-5121
• **BOLSTAD et al.** *Bioinformatics,* 2003, vol. 19, 185-193
• **SMYTH.** *Stat Appl Genet Mol Biol.,* 2004, vol. 3
• **STOREY ; TIBSHIRANI.** *Proc Natl Acad Sci USA.,* 2003, vol. 100, 9440-9445
• **STROCHOVA ; PELLMAN.** *Nat Rev Mol Cell. Biol.,* 2004, vol. 5